(19) Europäisches Patentamt
European Patent Office
Office européen des brevets

(11) Veröffentlichungsnummer : **0 036 390**
**B1**

(12) # EUROPÄISCHE PATENTSCHRIFT

(45) Veröffentlichungstag der Patentschrift : **26.10.83**

(21) Anmeldenummer : **81810092.7**

(22) Anmeldetag : **12.03.81**

(51) Int. Cl.³ : **C 07 C127/19**, C 07 D211/16, C 07 C147/06, C 07 C149/437, A 01 N 47/30

(54) **Diphenyläther-Harnstoffe mit herbizider Wirkung.**

(30) Priorität : **18.03.80 CH 2123/80**
**18.03.80 CH 2124/80**

(43) Veröffentlichungstag der Anmeldung : **23.09.81 Patentblatt 81/38**

(45) Bekanntmachung des Hinweises auf die Patenterteilung : **26.10.83 Patentblatt 83/43**

(84) Benannte Vertragsstaaten : **BE CH DE FR GB IT LI NL**

(56) Entgegenhaltungen :
**CH A 384 282**
**US A 3 707 557**

(73) Patentinhaber : **CIBA-GEIGY AG**
**Patentabteilung Postfach**
**CH-4002 Basel (CH)**

(72) Erfinder : **Rohr, Otto, Dr.**
**Kilbertweg 19**
**CH-4106 Therwil (CH)**
Erfinder : **Pissiotas, Georg, Dr.**
**Breslauerstrasse 8**
**D-7850 Lörrach (DE)**

**EP 0 036 390 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Diphenyläther-Harnstoffe mit herbizider Wirkung

Die vorliegende Erfindung betrifft neue Harnstoffe mit herbizider Wirkung, ihre Herstellung, sie enthaltende Mittel sowie deren Verwendung zur selektiven Bekämpfung von Unkräutern in verschiedenen Kulturen.

Aus vielen Patentanmeldungen ist bekannt, dass Diphenyläther und Phenylharnstoffe hervorragende Herbizide sind, z. B. USP 2 655 445, 2 655 447, 3 080 225 u. a.

Die Kombination Diphenyläther/Harnstoff ist ebenfalls z. B. aus der DT-OS 2 411 320, der BE-PS 623 440 und den CH-PS 384 282, 493 195 und 503 459 bekannt geworden. Ueberraschenderweise wurde nun gefunden, dass die neuen Derivate der vorliegenden Erfindung ein besonders gutes Verhältnis von Aktivität zu Selektivität aufweisen und dies vor allem bei einem postemergenten Einsatz beispielsweise in Kulturen wie Soja, Mais und Getreide.

Die neuen Verbindungen entsprechen der Formel I

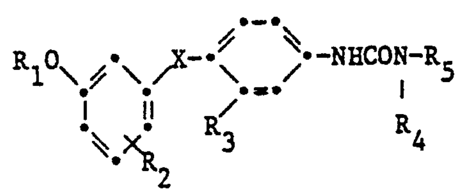

(I)

worin

$R_1$ einen $C_1$-$C_6$-Alkyl-, $C_2$-$C_6$-Alkenyl-, $C_2$-$C_6$-Alkinyl-, Aralkyl-, Phenyl-, $C_1$-$C_6$-Haloalkyl-, $C_1$-$C_6$-Cyanoalkyl-, $C_2$-$C_6$-Alkoxy-alkyl-, $C_2$-$C_6$-Alkylthioalkyl-, Acyl-, Haloacyl-, einen $C_1$-$C_4$-Alkylsulfonylrest oder eine Gruppe

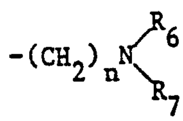

$R_2$ Wasserstoff, Halogen, $C_1$-$C_4$-Alkyl oder $C_1$-$C_4$-Alkoxy,

$R_3$ Wasserstoff, Halogen, Cyan oder Trifluormethyl,

$R_4$ Wasserstoff, $C_1$-$C_6$-Alkyl, $C_2$-$C_6$-Alkenyl, $C_2$-$C_6$-Alkinyl oder $C_1$-$C_6$-Alkoxy,

$R_5$ Wasserstoff, $C_1$-$C_6$-Alkyl, $C_2$-$C_6$-Alkenyl oder zusammen mit $R_4$ und dem Stickstoffatom, an das sie gebunden sind, auch einen 5-6-gliedrigen Heterocyclus,

$R_6$ und $R_7$ je einzeln Wasserstoff, $C_1$-$C_6$-Alkyl oder $C_2$-$C_6$-Alkenyl oder zusammen mit einem Stickstoffatom, an das sie gebunden sind, auch einen 5-6-gliedrigen Heterocyclus,

n eine Zahl 1, 2 oder 3 und

X Sauerstoff, Schwefel, die Sulfinyl- oder Sulfonylgruppe bedeuten.

In den obigen Definitionen kommt für « Alkyl » jeder geradkettige oder verzweigte Alkylrest in Frage, der bis zu 6 Kohlenstoffatome enthalten kann. Alkenylreste und Alkinylreste haben 2 bis 6 Kohlenstoffatome, können geradkettig oder verzweigt sein und eine oder zwei ungesättigte Stellen enthalten. Aralkylreste sind Phenylreste, die durch eine gerade oder verzweigte $C_1$-$C_6$-Alkylenkette an das Molekül gebunden sind. Phenylreste können unsubstituiert oder durch Halogen, $C_1$-$C_4$-Alkyl, $C_1$-$C_4$-Alkoxy, $C_1$-$C_4$-Alkylthio, Cyan, Nitro oder Trifluormethyl ein oder mehrfach substituiert sein. Acylreste $R_1$ sind Reste von Alkancarbonsäuren, die über die Esterfunktion an den Sauerstoff gebunden sind oder über die Hydroxygruppe gebundene Reste von Hydroxycarbonsäuren und deren Estern, wie z. B. Hydroxyessigsäure, α- oder β-Hydroxy-propionsäuren sowie α-, β- oder γ-Hydroxy-buttersäuren. Haloacylgruppen sind halogenierte Alkancarbonsäuren und deren Ester, wie beispielsweise die Chloressigsäure. Der 5-6-gliedrige Heterocyclus, den die Reste $R_4$ und $R_5$ respektive $R_6$ und $R_7$ zusammen mit dem Stickstoffatom, an das sie gebunden sind, bilden können, sind Pyrrolidin, Piperidin, Morpholin, Thiomorpholin oder Piperazinringe. Diese können durch Methyl substituiert sein, der Piperazinring auch durch Phenyl. Unter Halogen wird Fluor, Chlor, Brom und Jod verstanden, bevorzugt ist Chlor.

Die Herstellung der Verbindungen der Formel I geschieht in an sich bekannter, für Harnstoffe geeigneter Weise.

Das Verfahren zur Herstellung der Harnstoffe der Formel I ist dadurch gekennzeichnet, dass man ein Isocyanat der Formel II

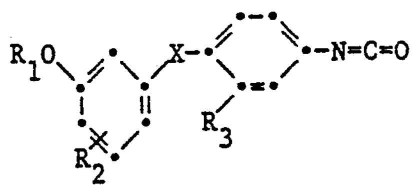

(II)

worin $R_1$, $R_2$ und $R_3$ die oben gegebene Bedeutung haben, in einem inerten organischen Lösungs- oder Verdünnungsmittel, mit einem Amin der Formel III umsetzt,

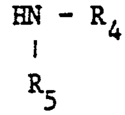

(III)

worin $R_4$ und $R_5$ die oben gegebenen Bedeutungen haben.

Diese Umsetzung kann bei 0-150 °C erfolgen, d. h. sie geht schon recht gut bei Raumtemperatur. Zwecks Verkürzung der Reaktionszeit wird im allgemeinen kurz auf Siedetemperatur erhitzt.

Als Lösungs- oder Verdünnungsmittel kommen für diese Umsetzung organische, mit Wasser mischbare organische Lösungsmittel in Frage, wie beispielsweise Aceton, Acetonitril, höhere Ketone, Dimethylformamid, Dimethylsulfoxyd, Dioxan etc. Im allgemeinen wird unter Normaldruck gearbeitet, grössere Mengen können auch in Druckgefässen hergestellt werden.

Die Ausgangsstoffe der Formel II, in denen X Sauerstoff bedeutet, werden z. B. durch folgende Reaktionssequenz hergestellt: Ein Phenol der Formel IV wird mit einem Halogen-Nitrobenzol der Formel V umgesetzt, wobei ein para-Nitrodiphenyläther der Formel VI entsteht. Dieser wird zum entsprechenden para-Diphenylätheramin der Formel VII reduziert, welcher seinerseits z. B. mittels Phosgen zum Isocyanat der Formel II umgewandelt wird.

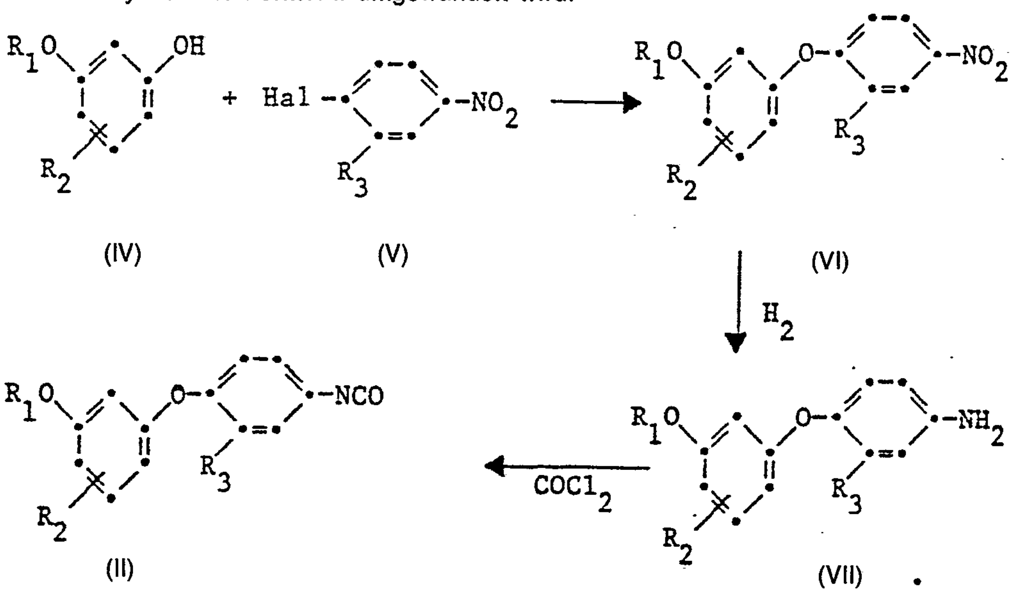


(IV) (V) (VI)

(II) (VII)

In den Formeln II bis VII haben $R_1$ und $R_2$ die oben gegebene Bedeutung, Hal bedeutet Halogen. Einzelne Nitrodiphenyläther der Formel VI sind in der JA-OS 73432/66 beschrieben. Andere Ausgangsstoffe der Formel II, können auf ähnliche Weise hergestellt werden.

Die Verbindungen der Formel I weisen ausgesprochen selektivherbizide Eigenschaften im allgemeinen auf und erweisen sich als besonders vorteilhaft zum Bekämpfen von Unkräutern in Kulturen von Nutzpflanzen, insbesondere in Kulturen von Soja, Getreide und Mais.

Bei genügend grosser Aufwandmenge ist jedoch auch totalherbizide Wirkung vorhanden. Die Anwendung kann sowohl im Vorauflaufwie im Nachauflaufverfahren vorgenommen werden. Dabei können die Aufwandmengen in weiten Grenzen schwanken, z. B. zwischen 0,1 bis 10 kg Wirkstoff pro Hektare, vorzugsweise werden jedoch 0,5 bis 5 kg Wirkstoff pro Hektare eingesetzt.

Die Verbindungen der Formel I eignen sich vor allem für den post-emergenten Einsatz als Selektivherbizide in Kulturen von Soja, Reis und Getreide. Bei einer Gegenüberstellung der Wirkung von erfindungsgemässen Verbindungen und derjenigen bekannter Herbizide in den obengenannten Kulturen, übertraf die Verbindung Nr. 1 die Wirkung der Handelsprodukte in Bezug auf Selektivität und Aktivität in den Kulturen Weizen und Soja, die Verbindung Nr. 39 war besser in der Kultur Weizen und die Verbindung Nr. 45 in der Kultur Reis. Auch die übrigen erfindungsgemässen Verbindungen stechen durch ihr überaus günstiges Verhältnis Aktivität/Selektivität hervor.

Besonders gute Wirkung zeigten die Harnstoffe der Formel Ia

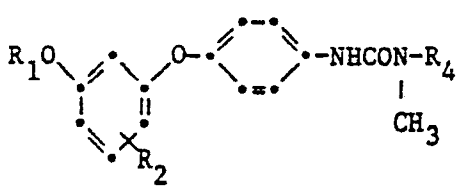

(Ia)

worin

$R_1$ einen gradkettigen oder verzweigten $C_1$-$C_6$-Alkylrest,

$R_2$ Wasserstoff, Halogen, Methyl oder Methoxy und

$R_4$ Methyl oder Methoxy bedeuten,

am besten haben diejenigen Verbindungen gewirkt, in denen $R_2$ Wasserstoff bedeutet.

Die Verbindungen der Formel I sind stabile Verbindungen. Sie sind für Warmblüter wenig giftig, ihre Handhabung bedarf keiner vorsorglicher Massnahmen. Sie sind in den üblichen organischen Lösungsmitteln relativ gut und in Wasser schlecht löslich. Sie können durch Zugeben von Wasser zur Reaktionslösung leicht ausgefällt werden. Ihre Formulierung als flüssige herbizide Mittel gelingt nur mit Hilfe besonderer Lösungsvermittler und/oder Dispergiermittel.

Erfindungsgemässe Mittel enthalten ausser dem Wirkstoff der Formel I einen geeigneten Träger und/oder andere Zuschlagstoffe. Geeignete Träger und Zuschlagstoffe können fest oder flüssig sein und entsprechen den in der Formulierungstechnik üblichen Stoffen.

Die Herstellung erfindungsgemässer Mittel erfolgt in an sich bekannter Weise durch inniges Vermischen und Vermahlen von Wirkstoffen der allgemeinen Formel I mit geeigneten Trägerstoffen und/oder Verteilungsmitteln, gegebenenfalls unter Zusatz von gegenüber den Wirkstoffen inerten Antischaum-, Netz-, Dispersions- und/oder Lösungsmitteln. Die Wirkstoffe können in den folgenden Aufarbeitungsformen vorliegen und angewendet werden :

Feste Aufarbeitungsformen : Stäubemittel, Streumittel, Granulate, Umhüllungsgranulate, Imprägniergranulate und Homogengranulate ;

In Wasser dispergierbare Wirkstoffkonzentrate : Spritzpulver (wettable powder), Pasten Emulsionen ; Emulsionskonzentrate ;

Flüssige Aufarbeitungsformen : Lösungen.

Die Wirkstoffkonzentrationen betragen in den erfindungsgemässen Mitteln 1 bis 80 Gewichtsprozent und können gegebenenfalls bei der Anwendung auch in geringen Konzentrationen wie etwa 0,05 bis 1 % vorliegen.

Den erfindungsgemässen Mitteln lassen sich andere biozide Wirkstoffe oder Mittel beimischen. So können die neuen Mitteln ausser den genannten Verbindungen der allgemeinen Formel I z. B. Insektizide, Fungizide, Bakterizide, Fungistatika, Bakteriostatika, Nematozide oder weitere Herbizide zur Verbreiterung des Wirkungsspektrums enthalten.

Die folgenden Beispiele sollen die Herstellung der erfindungsgemässen Diphenylätherharnstoffe der Formel I näher erläutern. Weitere in analoger Weise hergestellte Verbindungen sind in der sich dem Beispiel 2 anschliessenden Tabelle aufgeführt. Die Temperaturen sind in Celsiusgraden angegeben, Teile und Prozentangaben beziehen sich auf das Gewicht. Druckangaben sind in Millibar angegeben. In weiteren Beispielen wird die Aufbereitung der Wirkstoffe zu technisch verwendbaren Verabreichungsformen sowie Versuche zur Demonstration der herbiziden Wirkung beschrieben.

Beispiel 1

N-[4-(3'-Methoxyphenoxy)-phenyl]-N'-methoxy-N'-methylharnstoff

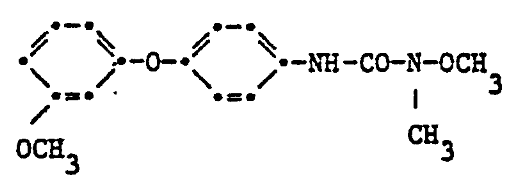

a) 124,1 g (1 Mol) 3-Methoxy-phenol und 44 g (1,1 Mol) pulverisiertes Natriumhydroxid werden in 700 ml Dimethylsulfoxid vorgelegt. Dazu tropft man 157,5 g p-Chlor-nitrobenzol, gelöst in 100 ml Dimethylsulfoxyd und erhitzt anschliessend über Nacht auf 120°. Das so erhaltene Reaktionsprodukt wird in Eis/Wasser gegossen und abfiltriert. Das Filtrat wird mit Wasser gewaschen und im Trockenschrank bei 50° getrocknet. Man erhält so 231 g 4-Nitro-3'-methoxydiphenyläther mit Smp. 82°-85°.

b) 98 g (0,4 Mol) 4-Nitro-3'-methoxydiphenyläther werden in Aethanol gelöst und nach Zugabe von Raney-Nickel hydriert. Nach beendeter Wasserstoffaufnahme wird der Katalysator abfiltriert und das Lösungsmittel abdestilliert. Das so erhaltene Oel wird im Vakuum destilliert und hat einen Siedepunkt von 143° bei 0,04 millibar.

c) Bei ca. – 20° wird in 250 ml Essigester 25 g Phosgen eingeleitet. Bei gleicher Temperatur tropft man nachher 43 g (0,2 Mol) des unter b) erhaltenen Amins zu, welches man zuvor in 100 ml Essigester gelöst hat. Die so erhaltene Mischung wird unter gleichzeitigem Einleiten von Phosgen im Verlaufe einer Stunde auf 70° erwärmt. Bei dieser Temperatur wird eine halbe Stunde weiter gerührt, anschliessend auf Raumtemperatur gekühlt und während 30 Minuten wird dann an Stelle von Phosgen Stickstoff durch die Lösung geleitet. Nach dem Abdestillieren des Lösungsmittels wird das so erhaltene Isocyanat roh weiter verarbeitet.

d) 18,3 g (0,3 Mol) Methoxy-methylamin werden in 100 ml Acetonitril gelöst. Anschliessend tropft

man unter gutem Rühren eine Lösung von 0,1 Mol des oben erhaltenen Isocyanates in 50 ml Acetonitril dazu. Das Reaktionsgemisch wird 30 Minuten weitergerührt und dann in Eis/Wasser gegossen. Die wässrige Mischung wird mit Aethylacetat/Toluol 1 : 1 extrahiert. Die organische Phase wird über Natriumsulfat getrocknet und dann wird das Lösungsmittel abgedampft. Man erhält so 30 g Titelverbindung als viskoses Oel mit dem Brechungsindex $n_D^{29}$ 1.5707.

Molekulargewicht :

Analyse C ber. 63,57 H ber. 6,00 N ber. 9,27 %

C gef. 64,3 H gef. 6,1 N gef. 8,9 %

Das NMR-Spektrum stimmt für die angenommene Struktur.

Beispiel 2

N-[4-(3'-α-Propionitriloxy-phenoxy)-phenyl]-N',N'-dimethylharnstoff

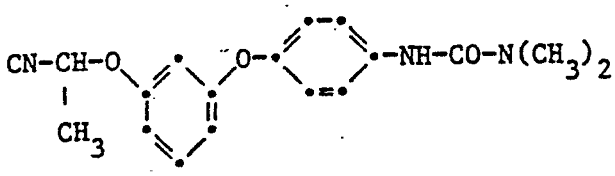

a) 23,1 g 4-Nitro-3'-Hydroxydiphenyläther und 15 g Brompropionsäurenitril werden über Nacht bei 80 °C in Aethyl-methylketon in Anwesenheit der 3-fachen Menge Kaliumcarbonat gerührt. Nach Abfiltrieren und Eidampfen erhält man 28 g Oel, 4-Nitro-3' (α-propionitriloxy)-diphenyläther.

b) Der unter a) erhaltene Diphenyläther wird nach Béchamp (mit Eisenfeilspänen und konzentrierter Salzsäure) hydriert und das rohe 4-Phenoxyanilin wird nach dem Isolieren mit Phosgen zum entsprechenden Isocyanat umgesetzt.

0,05 Mol des so erhaltenen Isocyanats werden in 50 ml Acetonitril gelöst und langsam in eine Lösung von 6 g Dimethylamin in 100 ml Acetonitril getropft. Nach beendeter Reaktion versetzt man mit Wasser und filtriert die ausgeschiedenen Kristalle ab. Man erhält nach dem Trocknen im Vakuum 10,6 g der Titelverbindung als weisses Pulver vom Schmelzpunkt 105-111°. (Verbindung No. 25)

Auf analoge Weise werden folgende Verbindungen hergestellt.

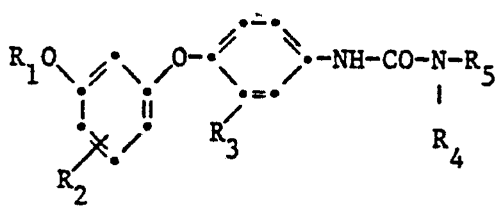

| No. | $R_1O$- | $R_2$ | $R_3$ | $R_4$ | $R_5$ | phys. Daten |
|---|---|---|---|---|---|---|
| 1 | $CH_3O$ | - | - | $OCH_3$ | $CH_3$ | $n_D^{29}$ 1.5707 |
| 2 | $CH_3O$ | - | - | $CH_3$ | $CH_3$ | Smp. 139 - 141° |
| 3 | $CH_3O$ | 6-Cl | - | $CH_3$ | $CH_3$ | Smp- 141-143° |
| 4 | $CH_3O$ | 6-Cl | - | $OCH_3$ | $CH_3$ | Smp. 96 - 97° |
| 5 | $CH_3O$ | 5-$OCH_3$ | - | $CH_3$ | $CH_3$ | Smp. 140-141° |
| 6 | $CH_3O$ | 5-$OCH_3$ | - | $OCH_3$ | $CH_3$ | Smp. 99-100° |
| 7 | $CH_3O$ | 4-Cl | - | $CH_3$ | $CH_3$ | Smp. 139 - 140° |
| 8 | $CH_3O$ | 4-Cl | - | $OCH_3$ | $CH_3$ | Smp. 93 - 94° |
| 9 | $C_2H_5O$ | - | - | $OCH_3$ | $CH_3$ | Oel |
| 10 | $C_2H_5O$ | - | - | $CH_3$ | $CH_3$ | Smp. 121-128° |

(Fortsetzung)

| No. | $R_1O$ | $R_2$ | $R_3$ | $R_4$ | $R_5$ | phys. Daten |
|---|---|---|---|---|---|---|
| 11 | $C_2H_5O$ | 6-Cl | - | $CH_3$ | $CH_3$ | |
| 12 | $C_2H_5O$ | 6-Cl | - | $OCH_3$ | $CH_3$ | |
| 13 | $C_2H_5O$ | 4-Br | - | $CH_3$ | $CH_3$ | |
| 14 | $C_2H_5O$ | 4-Br | - | $OCH_3$ | $CH_3$ | |
| 15 | $C_2H_5O$ | 4-Cl | - | $CH_3$ | $CH_3$ | |
| 16 | $C_2H_5O$ | 4-Cl | - | $OCH_3$ | $CH_3$ | |
| 17 | $OC_3H_7$ iso | - | - | $CH_3$ | $CH_3$ | Smp. 92-96° |
| 18 | $OC_3H_7$ iso | - | - | $OCH_3$ | $CH_3$ | $n_D^{26}$ 1.5692 |
| 19 | $OC_3H_7$ iso | 6-Cl | - | $CH_3$ | $CH_3$ | |
| 20 | $OC_3H_7$ iso | 6-Cl | - | $OCH_3$ | $CH_3$ | |
| 21 | $OC_3H_7$ iso | 4-Br | - | $CH_3$ | $CH_3$ | |
| 22 | $OC_3H_7$ iso | 4-Br | - | $OCH_3$ | $CH_3$ | |
| 23 | $OC_3H_7$ iso | 4-Cl | - | $CH_3$ | $CH_3$ | |
| 24 | $OC_3H_7$ iso | 4-Cl | - | $OCH_3$ | $CH_3$ | |
| 25 | $OCH(CH_3)CN$ | - | - | $CH_3$ | $CH_3$ | Smp. 105-111° |
| 26 | $OCH(CH_3)CN$ | - | - | $OCH_3$ | $CH_3$ | Oel |
| 27 | $OCH_2$- 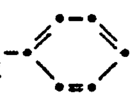 | - | - | $CH_3$ | $CH_3$ | Smp. 85-87° |
| 28 | $OCH_2$- 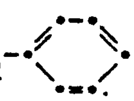 | - | - | $OCH_3$ | $CH_3$ | Smp. 95-96° |
| 29 | $OCOCH_3$ | - | - | $CH_3$ | $CH_3$ | Smp. 137-139° |
| 30 | $OCOCH_3$ | - | - | $OCH_3$ | $CH_3$ | Oel |
| 31 | $OCH_3$ | | | | | |
| 32 | $OCH(CH_3)CN$ | 4-Cl | - | $CH_3$ | $CH_3$ | Smp. 146-148° |
| 33 | $OCH(CH_3)CN$ | 4-Cl | - | $OCH_3$ | $CH_3$ | Smp. 118-119° |
| 34 | $OCH(C_2H_5)CN$ | 4-Cl | - | $CH_3$ | $CH_3$ | Smp. 104-110° |

(Fortsetzung)

| No. | $R_1O$ | $R_2$ | $R_3$ | $R_4$ | $R_5$ | phys. Daten |
|---|---|---|---|---|---|---|
| 35 | $OCH(C_2H_5)CN$ | 4-Cl | - | $OCH_3$ | $CH_3$ | Oel |
| 36 | $OCH(CH_3)CN$ | 6-Cl | - | $CH_3$ | $CH_3$ | Smp. 125-128° |
| 37 | $OCH(CH_3)CN$ | 6-Cl | - | $OCH_3$ | $CH_3$ | Oel |
| 38 | $OCH_3$ | - | Cl | $CH_3$ | $CH_3$ | Smp. 168-170° |
| 39 | $OCH_3$ | - | Cl | $OCH_3$ | $CH_3$ | $n_D^{26}$ 1.5843 |
| 40 | $OCH(CH_3)CN$ | - | Cl | $CH_3$ | $CH_3$ | Smp. 80-84° |
| 41 | $OCH(CH_3)CN$ | - | Cl | $OCH_3$ | $CH_3$ | Oel |
| 42 | $OCH_3$ | 6-Cl | Cl | $CH_3$ | $CH_3$ | Smp. 164-170° |
| 43 | $OCH_3$ | 6-Cl | Cl | $OCH_3$ | $CH_3$ | Smp. 91-94° |
| 44 | $OCH_3$ | 4-Cl | Cl | $CH_3$ | $CH_3$ | Smp. 177-178° |
| 45 | $OCH_3$ | 4-Cl | Cl | $OCH_3$ | $CH_3$ | Smp. 117-118° |
| 46 | $OCH_3$ | 4-Cl | Cl | $OC_4H_{9n}$ | $CH_3$ | Smp. 137-138° |
| 47 | $OCH(CH_3)CN$ | 4-Cl | Cl | $CH_3$ | $CH_3$ | glasartig |
| 48 | $OCH(CH_3)CN$ | 4-Cl | Cl | $OCH_3$ | $CH_3$ | glasartig |
| 49 | $OCH(CH_3)CN$ | 6-Cl | Cl | $CH_3$ | $CH_3$ | Oel |
| 50 | $OCH(CH_3)CN$ | 6-Cl | Cl | $OCH_3$ | $CH_3$ | Oel |
| 51 | $OCH_3$ | 4,6-$Cl_2$ | Cl | $CH_3$ | $CH_3$ | Smp. 193-195° |
| 52 | $OCH_3$ | 4,6-$Cl_2$ | Cl | $OCH_3$ | $CH_3$ | Smp. 139-141° |
| 53 | $OCH_3$ | - | $CF_3$ | $CH_3$ | $CH_3$ | Smp. 104-106° |
| 54 | $OCH_3$ | - | $CF_3$ | $OCH_3$ | $CH_3$ | Smp. 78-79° |
| 55 | $OCH_3$ | 4-Cl | $CF_3$ | $CH_3$ | $CH_3$ | Smp. 121-125° |
| 56 | $OCH_3$ | 4-Cl | $CF_3$ | $OCH_3$ | $CH_3$ | $n_D^{25}$ 1.5500 |
| 57 | $OCH_3$ | 6-Cl | $CF_3$ | $CH_3$ | $CH_3$ | Smp. 157-161° |
| 58 | $OCH_3$ | 6-Cl | $CF_3$ | $OCH_3$ | $CH_3$ | Smp. 109-111° |
| 59 | $OCH(CH_3)COOCH_3$ | 4-Cl | Cl | $CH_3$ | $CH_3$ | Smp. 136-137° |

(Fortsetzung)

| No. | $OR_1$ | $R_2$ | $R_3$ | $R_4$ | $R_5$ | phys. Daten |
|---|---|---|---|---|---|---|
| 60 | $OCH(CH_3)COOCH_3$ | 4-Cl | Cl | $OCH_3$ | $CH_3$ | Smp. 82-86° |
| 61 | $OCH(CH_3)COOCH_3$ | 4-Cl | Cl | $OC_4H_{9n}$ | $CH_3$ | Smp. 84-88° |
| 62 | $OCH_3$ | - | - | $CH_3$ | H | |
| 63 | $OCH_3$ | - | - | $CH(CH_3)C{\equiv}CH$ | $CH_3$ | Smp. 175-176° |
| 64 | $OCH_3$ | - | - | 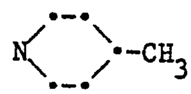 | | Smp. 128-129° |
| 65 | $OCH_3$ | - | - | $C_4H_{9n}$ | $CH_3$ | Smp. 71-72° |
| 66 | $OCH_3$ | - | - | $C_2H_5$ | $CH_3$ | Smp. 93-94° |
| 67 | $OCH_3$ | - | - | $C_3H_7$iso | $CH_3$ | Smp. 157-158° |
| 68 | $OCOCH_2Cl$ | - | - | $CH_3$ | $CH_3$ | Smp. 84-85° |
| 69 | $OCOCH_2Cl$ | - | - | $OCH_3$ | $CH_3$ | Oel |
| 70 | $OCOCHCl_2$ | - | - | $CH_3$ | $CH_3$ | Smp. 82-83° |
| 71 | $OCOCHCl_2$ | - | - | $OCH_3$ | $CH_3$ | Oel |
| 72 | 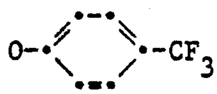 | - | - | $CH_3$ | $CH_3$ | |
| 73 | 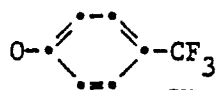 | - | - | $OCH_3$ | $CH_3$ | |
| 74 | OCONH- 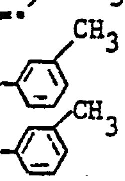 | - | - | $CH_3$ | $CH_3$ | Smp. 83-84° |
| 75 | OCONH- | - | - | $OCH_3$ | $CH_3$ | Wachs |
| 76 | $OCONHCH_3$ | - | - | $CH_3$ | $CH_3$ | Smp. 135-139° |
| 77 | $OCONHCH_3$ | - | - | $OCH_3$ | $CH_3$ | Smp. 123-126° |
| 78 | $OCH_2CH{=}CH_2$ | - | - | $CH_3$ | $CH_3$ | Smp. 83-85° |
| 79 | $OCH_2CH{=}CH_2$ | - | - | $OCH_3$ | $CH_3$ | Smp. 47-51° |
| 80 | OCONH- 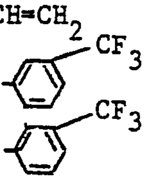 | - | - | $CH_3$ | $CH_3$ | Smp. 124-125° |
| 81 | OCONH- | - | - | $OCH_3$ | $CH_3$ | Smp. 192-194° |
| 82 | $OC_4H_{9n}$ | - | - | $CH_3$ | $CH_3$ | Smp. 87-90° |

(Fortsetzung)

| No. | $OR_1$ | $R_2$ | $R_3$ | $R_4$ | $R_5$ | phys. Daten |
|---|---|---|---|---|---|---|
| 83 | $OC_4H_{9n}$ | - | - | $OCH_3$ | $CH_3$ | Oel |
| 84 | $OCH_2CH{=}CHClCH_3$ | - | - | $CH_3$ | $CH_3$ | |
| 85 | $OCH_2CH{=}CHClCH_3$ | - | - | $OCH_3$ | $CH_3$ | |
| 86 | $OCH_2CCl{=}CH_2$ | - | - | $CH_3$ | $CH_3$ | |
| 87 | $OCH_2CCl{=}CH_2$ | - | - | $OCH_3$ | $CH_3$ | |
| 88 | $OCH_2C{\equiv}CH$ | - | - | $CH_3$ | $CH_3$ | Smp. 110-116° |
| 89 | $OCH_2C{\equiv}CH$ | - | - | $OCH_3$ | $CH_3$ | Oel |
| 90 | $OCH(CH_3)COOCH_3$ | - | - | $CH_3$ | $CH_3$ | Smp. 102-105° |
| 91 | $OCH(CH_3)COOCH_3$ | - | - | $OCH_3$ | $CH_3$ | Oel |
| 92 | $OCOCF_3$ | - | - | $CH_3$ | $CH_3$ | Smp. 85-87° |
| 93 | $OCOCF_3$ | - | - | $OCH_3$ | $CH_3$ | Smp. 114-115° |
| 94 | $OCH_2$- 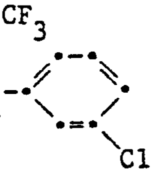 | - | - | $CH_3$ | $CH_3$ | Smp. 85-87° |
| 95 | $OCH_2$- 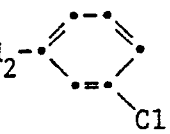 | - | - | $OCH_3$ | $CH_3$ | Smp. 95-96° |
| 96 | $OCH_2$- 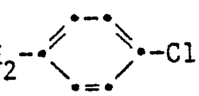 | - | - | $CH_3$ | $CH_3$ | |
| 97 | $OCH_2$- 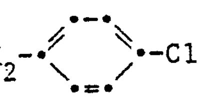 | - | - | $OCH_3$ | $CH_3$ | |
| 98 | $OSO_2CH_3$ | - | - | $CH_3$ | $CH_3$ | Smp. 101-103° |
| 99 | $OSO_2CH_3$ | - | - | $OCH_3$ | $CH_3$ | Smp. 64-68° |
| 100 | $OCONHC_2H_4Cl$ | - | - | $CH_3$ | $CH_3$ | Smp. 100-110° |
| 101 | $OCONHC_2H_4Cl$ | - | - | $OCH_3$ | $CH_3$ | Oel |
| 102 | $OCH_3$ | - | - | $(CH_2CH{=}CH_2)_2$ | | Smp. 88-89° |
| 103 | $OCH_3$ | - | - | CHO | $CH_3$ | Oel |
| 104 | $OCH(CH_3)CH_2OH$ | 4-Cl | Cl | $OCH_3$ | $CH_3$ | Smp. 97-100° |

(Fortsetzung)

| No. | $OR_1$ | $R_2$ | X | $R_3$ | $R_4$ | $R_5$ | phys. Daten |
|---|---|---|---|---|---|---|---|
| 105 | $OCH_3$ | - | | CN | $CH_3$ | $CH_3$ | Smp. 103-105° |
| 106 | $OCH_3$ | - | | CN | $OCH_3$ | $CH_3$ | Smp. 104-105° |
| 107 | $OCONHC_4H_9$t.. | - | | - | $C_4H_9$t. | H | Smp. 65-67° |
| 108 | $OCH(CH_3)CSNH_2$ | - | | - | $CH_3$ | $CH_3$ | |
| 109 | $OCH(CH_3)CSNH_2$ | - | | - | $OCH_3$ | $CH_3$ | |
| 110 | $OCH(CH_3)CSN{=}CHN(CH_3)_2$ | - | | - | $CH_3$ | $CH_3$ | |
| 111 | $OCH(CH_3)CSN{=}CHN(CH_3)_2$ | - | | - | $OCH_3$ | $CH_3$ | |

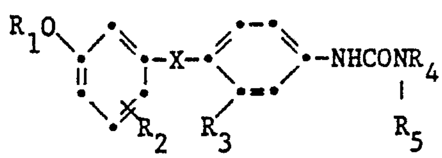

| No. | $OR_1$ | $R_2$ | X | $R_3$ | $R_4$ | $R_5$ | phys. Daten |
|---|---|---|---|---|---|---|---|
| 112 | $OCH_3$ | - | S | - | $CH_3$ | $CH_3$ | Smp. 128-129° |
| 113 | $OCH_3$ | - | S | - | $OCH_3$ | $CH_3$ | Smp. 79-81° |
| 114 | $OCH_3$ | - | $SO_2$ | - | $CH_3$ | $CH_3$ | Smp. 129-140° |
| 115 | $OCH_3$ | - | $SO_2$ | - | $OCH_3$ | $CH_3$ | Smp. 120-121° |

Beispiel 3

Das Aufbereiten der Verbindungen der Formel I in für die Landwirtschaft brauchbare Anwendungsformen kann beispielsweise gemäss einer der folgenden Vorschriften erfolgen :

Spritzpulver

Zur Herstellung eines a) 70 %igen und b) 10 %igen Spritzpulvers werden folgende Bestandteile verwendet :

a) 70 Teile N-[4-(3'-Methoxyphenoxy)-phenyl]-N',N'-dimethylharnstoff
   5 Teile Natriumdibutylnaphthylsulfonat,
   3 Teile Naphthalinsulfonsäuren-Phenolsulfonsäuren-Formaldehyd-Kondensat 3 : 2 : 1,
   10 Teile Kaolin,
   12 Teile Kreidepulver

b) 10 Teile Wirkstoff
   3 Teile Gemisch der Natriumsalze von gesättigten Fettalkoholsulfaten,
   5 Teile Naphthalinsulfonsäuren-Formaldehyd-Kondensat,
   82 Teile Kaolin.

Der angegebene Wirkstoff wird auf die entsprechenden Trägerstoffe (Kaolin und Kreide) aufgezogen und anschliessend vermischt und vermahlen mit den übrigen Bestandteilen. Man erhält Spritzpulver von vorzüglicher Benetzbarkeit und Schwebefähigkeit. Aus solchen Spritzpulvern können durch Verdünnen mit Wasser Suspensionen von 0,1-8 % Wirkstoff erhalten werden, die sich zur Unkrautbekämpfung in Pflanzenkulturen eignen.

Emulsionskonzentrat

Zur Herstellung eines 25 %igen Emulsionskonzentrates werden

25 Teile N-[4-(3'-Methoxyphenoxy)-phenyl]-N'-methyl-N'-methoxyharnstoff
10 Teile einer Mischung von Nonylphenolpolyoxyäthylen und Calciumdodecylbenzolsulfonat,
10 Teile Cyclohexanon
55 Teile Xylol

miteinander vermischt. Dieses Konzentrat kann mit Wasser zu Emulsionen auf geeignete Konzentrationen von z. B. 0,1 % verdünnt werden. Solche Emulsionen eignen sich zur Bekämpfung von Unkräutern in Kulturpflanzungen.

Paste

Zur Herstellung einer 45 %igen Paste werden folgende Stoffe verwendet :

a) 45 Teile N-[4-(3'-Methoxyphenoxy)-phenyl]-N',N'-dimethyl-harnstoff
5 Teile Natriumaluminiumsilikat,
14 Teile Cetylpolyglykoläther mit 8 Mol Aethylenoxid,
1 Teil Oleylpolyglykoläther mit 5 Mol Aethylenoxid,
2 Teile Spindelöl,
10 Teile Polyäthylenglykol,
23 Teile Wasser.

b) 45 Teile N-[4-(3'-Methoxyphenoxy)-phenyl]-N'-methyl-N'-methoxy-harnstoff
5 Teile Aethylenglycol
3 Teile Octylphenoxypolyäthylenglycol mit 9-10 Mol Aethylenoxyd pro Mol Octylphenol
3 Teile von einem Gemisch aromatischer Sulfonsulfosäuren, kondensiert mit Formaldehyd als Ammoniumsalz
1 Teil Siliconöl in Form einer 75 %igen Emulsion
0,1 Teil einer Mischung von 1-(3-Chlorallyl) 3,5,7-triazonium-adamantan-chlorid mit Natriumcarbonat, Chloridwert mind. 11,5 %
0,2 Teile eines biopolymeren Verdickers mit max. 100 Keimen pro Gramm
42,7 Teile Wasser

Die Aktivsubstanz wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

Beispiel 4

Die herbizide Wirkung der Verbindungen der Formel I ist durch folgende Versuche ermittelt worden.

Pre-emergente Herbizid-Wirkung (Keimhemmung)

Im Gewächshaus werden Pflanzensamen in Blumentöpfe von 12-15 cm Durchmesser gesät. Unmittelbar danach wird die Erdoberfläche mit einer wässrigen Dispersion der Testverbindungen, erhalten aus einem Emulsionskonzentrat, Flowable oder Spritzpulver, behandelt. Es werden verschiedene Konzentrationsreihen angewendet und die Menge Wirkstoff die aufgetragen wurde in kg pro Hektar ausgedrückt. Die Töpfe werden dann im Gewächshaus bei einer Temperatur von 22-25° und 50-70 % rel. Luftfeuchtigkeit gehalten und der Versuch nach 3 Wochen ausgewertet.

In diesem Versuch zeigten die Verbindungen 1-8 in Aufwandmengen von weniger als 1 kg/ha gute Herbizidwirkung.

Post-emergente Herbizid Wirkung (Kontaktherbizid)

Eine grössere Anzahl Unkräuter und Kulturpflanzen, sowohl monocotyle wie dicotyle, wurden nach dem Auflaufen (im 4-bis-6-Blattstadium) mit einer wässrigen Wirkstoffdispersion in Dosierungen von 2

und 1 kg pro Hektar Wirksubstanz auf die Pflanzen gespritzt und diese bei 24°-26 °C und 45-60 % relativer Luftfeuchtigkeit gehalten. 15 Tage nach der Behandlung wird der Versuch ausgewertet und die Resultate nach folgender Notenskala (EWRC-Wertung) bonitiert : (European Weed Research Council)

1 = Pflanzen nicht gekeimt oder total abgestorben
2-3 = sehr starke Wirkung
4-6 = mittlere Wirkung
7-8 = geringe Wirkung
9 = keine Wirkung (wie unbehandelte Kontrolle)
— = Pflanze nicht geprüft.

Die Resultate sind in der folgenden Tabelle wiedergegeben

| Verbindung No. | 1 | 17 | 18 | 34 | 39 | 53 | 56 | 113 |
|---|---|---|---|---|---|---|---|---|
| Aufwandmenge kg/ha | 2 1 | 2 1 | 2 1 | 2 1 | 2 1 | 2 1 | 2 1 | 2 1 |
| Pflanze | | | | | | | | |
| Gerste | 5 9 | - - | 7 9 | 8 8 | 4 5 | - - | - - | 7 9 |
| Weizen | 4 9 | 9 9 | 7 9 | 8 9 | 9 9 | 9 9 | 7 8 | 8 9 |
| abutilon sp. | 1 1 | 1 1 | 1 1 | 1 1 | 1 1 | 1 1 | 1 1 | 1 1 |
| sida spinosa | 1 1 | - - | 4 6 | 5 5 | 1 1 | - - | - - | 1 2 |
| xanthium | - - | 1 2 | 1 1 | - - | - - | 1 1 | 1 1 | 4 7 |
| amarantus retro-flexus | 1 1 | - - | 1 2 | 1 2 | 1 1 | - - | - - | 1 1 |
| chenopodium album | 1 2 | 1 3 | 1 1 | - - | - - | 1 1 | 1 1 | 1 2 |
| solanum nigrum | 1 1 | - - | 2 2 | 1 2 | 1 1 | - - | - - | 1 1 |
| ipomoea purp. | 1 2 | 2 3 | 1 1 | 2 3 | 1 1 | 1 3 | 2 3 | 3 4 |
| sinapis alba | 1 1 | 1 1 | 1 1 | 1 1 | 1 1 | 1 2 | 1 2 | 1 4 |
| stellaria media | 1 2 | - - | 1 1 | 2 4 | 1 1 | - - | - - | 1 2 |
| galium aparine | 1 2 | 1 1 | 1 1 | 3 4 | 1 2 | 5 5 | 4 5 | 3 4 |
| viola tricolor | 2 2 | 1 1 | 1 2 | - - | - - | 2 3 | 2 3 | 2 2 |
| veronica | 1 1 | - - | 1 1 | - - | - - | - - | - - | 1 1 |
| kochi | - - | - - | - - | 3 3 | 1 1 | - - | - - | - - |
| portulacca | 1 1 | - - | - - | 1 2 | 1 1 | - - | - - | - - |
| sesbania exaltata | 1 2 | - - | - - | 1 1 | 1 1 | - - | - - | - - |

Selektive herbizide Wirkung auf Soja

Im Gewächshaus werden die Testpflanzen in Blumentöpfen mit sterilisierter Gartenerde angesät. Wenn sie das 4-6-Blatt-stadium erreicht haben, wird der Wirkstoff als wässrige Emulsion auf die Pflanzen in solcher Weise appliziert, dass es einer Aufwandmenge von 1 kg pro Hektar Wirkstoff im Feld entspricht. Die Töpfe werden dann in einem klimatisierten Gewächshaus bei 22-25° und 60-70 % relativer Luftfeuchtigkeit aufgestellt und jeden Tag begossen. Die Kontrolle und Beurteilung des Wachstums erfolgt nach 3 Wochen und der Zustand der Pflanzen wird nach dem obigen Index bewertet.

Resultate bei einer Aufwandmenge von 1 kg/ha sind wie folgt :

| Verbindung Nr. | 1 | 2 | 7 | 8 | 18 | 26 | 40 | 45 | 48 |
|---|---|---|---|---|---|---|---|---|---|
| Pflanze Soja | 7 | 9 | 7 | 7 | 6 | 6 | 6 | 8 | 8 |
| digitaria sanguinalis | 1 | 4 | 9 | 9 | 2 | 3 | 2 | 6 | 9 |
| sida spinosa | 1 | 4 | 6 | 6 | 6 | 9 | 4 | 4 | 3 |
| amarantus retroflexus | 1 | 1 | 2 | 2 | 2 | 2 | 2 | 2 | 4 |
| iopomoea purpurea | 1 | 4 | 2 | 2 | 1 | 3 | 3 | 5 | 6 |
| abutilon sp. | 1 | 3 | 2 | 2 | 1 | 1 | 7 | 7 | 1 |

Selektive herbizide Wirkung auf Reis im Nachauflaufverfahren

Reispflänzchen, welche 25 Tage alt sind, wurden im Gewächshaus in grosse rechteckige Eternitschalen verpflanzt. Zwischen die Reihen der Reispflanzen wurden dann Samen der in Reiskulturen vorkommenden Unkräuter echinochloa crus galli, cyperus difformis, ammania indica und rotala indica gesät. Die Schalen wurden gut bewässert und bei einer Temperatur von ca. 25 °C und hoher Luftfeuchtigkeit gehalten. Nach 12 Tagen, wenn die Unkräuter aufgelaufen sind und das 2-3 Blattstadium erreicht haben, wurde die Erde in der Schale mit einer 2,5 cm hohen Schicht Wasser bedeckt. Der Wirkstoff wurde dann als Emulsionskonzentrat mittels einer Pipette zwischen die Pflanzenreihen appliziert, wobei man das Emulsionskonzentrat so verdünnte und auftrug, dass es einer Applikationsmenge im Feld von 4 und 2 kg Wirkstoff pro Hektar entsprach. Der Versuch wurde nach 4 Wochen ausgewertet und der Zustand der Pflanzen nach dem obigen Bewertungsschema beurteilt. Die Resultate waren wie folgt:

| Pflanze | Reis | | echinochloa crus galli | | cyperus difformis | | ammania indica | | rotala indica | |
|---|---|---|---|---|---|---|---|---|---|---|
| Aufwandmenge kg/ha | 4 | 2 | 4 | 2 | 4 | 2 | 4 | 2 | 4 | 2 |
| Verbindung Nr. | | | | | | | | | | |
| 2 | 4 | 6 | 1 | 1 | 1 | 1 | 1 | 1 | 1 | 1 |
| 3 | 9 | 9 | 2 | 3 | 1 | 2 | 2 | 2 | 7 | 8 |
| 5 | 6 | 7 | 2 | 6 | 2 | 3 | 1 | 2 | 1 | 2 |
| 18 | 9 | 9 | 7 | 8 | 1 | 4 | 1 | 2 | 6 | 7 |
| 39 | 4 | 7 | 2 | 6 | 1 | 1 | 1 | 1 | 2 | 2 |
| 41 | 8 | 9 | 4 | 8 | 1 | 2 | 1 | 1 | 1 | 1 |
| 45 | 8 | 9 | 1 | 3 | 3 | 3 | 1 | 1 | 1 | 1 |

Desiccations- und Defoliationswirkung

In einem Gewächshaus wurden Baumwollpflanzen der Sorte Deltapine in Tontöpfen angezogen. Nach abgeschlossener Kapselbildung wurden sie mit wässerigen Zubereitungen des Wirkstoffes No. 1 in einer Aufwandmenge, die 1,2, 0,6 und 0,3 kg/ha im Feld entspräche, gespritzt. Unbehandelte Pflanzen wurden als Kontrolle belassen. Die Auswertung des Versuches erfolgte 3, 7 und 14 Tage nach Applikation der Wirksubstanz durch Bestimmen des Grades an Defoliation (% abgefallene Blätter) und an Dessikation (% Austrocknung der an der Pflanze verbleibenden Blätter).

In diesem Versuch beliessen die Verbindungen Nr. 1, 17, 18 und 113 bei Aufwandmengen von 0,6 und

1,2 kg/ha nach 7 Tagen bloss noch wenige vertrocknete Blätter auf den Pflanzen (> 80 % Blattfall und Austrocknung).

## Ansprüche

1. Neue Harnstoffe der Formel I

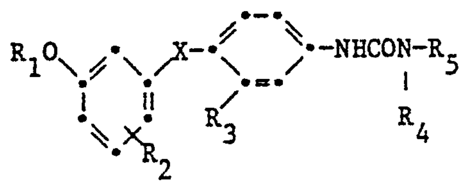

(I)

worin

$R_1$ einen $C_1$-$C_6$-Alkyl-, $C_2$-$C_6$-Alkenyl-, $C_2$-$C_6$-Alkinyl-, Aralkyl-, Phenyl-, $C_1$-$C_6$-Haloalkyl-, $C_1$-$C_6$-Cyanoalkyl-, $C_2$-$C_6$-Alkoxyalkyl-, $C_2$-$C_6$-Alkylthioalkyl-, Acyl-, Haloacyl-, einen $C_1$-$C_4$-Alkylsulfonylrest oder eine Gruppe

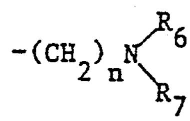

$R_2$ Wasserstoff, Halogen, $C_1$-$C_4$-Alkyl oder $C_1$-$C_4$-Alkoxy,

$R_3$ Wasserstoff, Halogen, Cyan oder Trifluormethyl,

$R_4$ Wasserstoff, $C_1$-$C_4$-Alkyl, $C_2$-$C_6$-Alkenyl, $C_2$-$C_6$-Alkinyl oder $C_1$-$C_6$-Alkoxy,

$R_5$ Wasserstoff, $C_1$-$C_6$-Alkyl, $C_2$-$C_6$-Alkenyl oder zusammen mit $R_4$ und dem Stickstoffatom, an das sie gebunden sind, auch einen 5-6-gliedrigen Heterocyclus,

$R_6$ und $R_7$ je einzeln Wasserstoff, $C_1$-$C_6$-Alkyl oder $C_2$-$C_6$-Alkenyl oder zusammen mit dem Stickstoffatom, an das sie gebunden sind, auch einen 5-6-gliedrigen Heterocyclus,

n eine Zahl 1, 2 oder 3 und

X Sauerstoff, Schwefel, die Sulfinyl- oder Sulfonylgruppe

bedeuten.

2. Die Verbindungen gemäss Anspruch 1 entsprechend der Formel I, worin X Sauerstoff und $R_3$ Wasserstoff bedeuten, während $R_1$, $R_2$, $R_4$ und $R_5$ die gegebene Bedeutung haben.

3. Die Verbindungen gemäss Anspruch 1 der Formel Ia

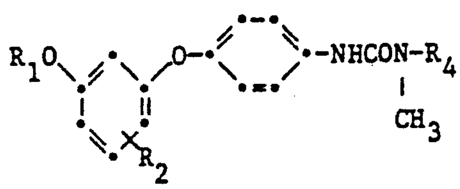

(Ia)

worin

$R_1$ einen geradkettigen oder verzweigten $C_1$-$C_6$-Alkylrest,

$R_2$ Wasserstoff, Halogen, Methyl oder Methoxy, und

$R_4$ Methyl oder Methoxy bedeuten.

4. Die Verbindungen gemäss Anspruch 1 der Formel Ib

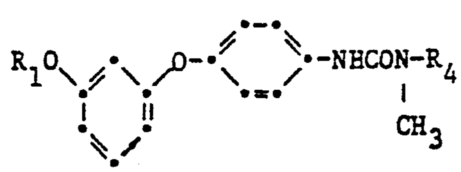

(Ib)

worin

$R_1$ einen geradkettigen oder verzweigten $C_1$-$C_6$-Alkylrest und

$R_4$ Methyl oder Methoxy bedeuten.

5. Die Verbindungen gemäss Anspruch 1 entsprechend der Formel I, worin X Schwefel, eine Sulfinyl-

oder Sulfonylgruppe, $R_2$ und $R_3$ je Wasserstoff bedeuten, während $R_1$, $R_4$ und $R_5$ die gegebene Bedeutung haben.

6. Als Verbindung gemäss Anspruch 1 N-[4-(3'-Methoxyphenoxy)-phenyl]-N',N'-dimethyl-harnstoff.

7. Als Verbindung gemäss Anspruch 1 N-[4-(3'-Methoxyphenoxy)-phenyl]-N'-methyl-N'-methoxy-harnstoff.

8. Als Verbindung gemäss Anspruch 1 N-[3-Chlor-4-(3'-methoxy-phenoxy)-phenyl]-N',N'-dimethyl-harnstoff.

9. Als Verbindung gemäss Anspruch 1 N-[3-Chlor-4-(3'-methoxy-phenoxy)-phenyl]-N'-methyl-N'-methoxy-harnstoff.

10. Als Verbindung gemäss Anspruch 1 N-[4-(3',5'-Dimethoxyphenoxy)-phenyl]-N',N'-dimethyl-harnstoff.

11. Als Verbindung gemäss Anspruch 1 N-[4-(3',5'-Dimethoxyphenoxy)-phenyl]-N'-methyl-N'-methoxy-harnstoff.

12. Als Verbindung gemäss Anspruch 1 N-[3-Chlor-4-(4'-chlor-3'-methoxyphenoxy)-phenyl]-N',N'-dimethyl-harnstoff.

13. Als Verbindung gemäss Anspruch 1 N-[3-Chlor-4-(4'-chlor-3'-methoxyphenoxy)-phenyl]-N'-methyl-N'-methoxy-harnstoff.

14. Als Verbindung gemäss Anspruch 1 N-[4-(3'-Methoxy-6'-chlor-phenoxy)-phenyl]-N',N'-dimethylharnstoff.

15. Als Verbindung gemäss Anspruch 1 N-[4-(3'-Propargyloxy-phenoxy)-phenyl]-N',N'-dimethylharnstoff.

16. Als Verbindung gemäss Anspruch 1 N-[4-(3'-Propargyloxy-phenoxy)-phenyl]-N'-methyl-N'-methoxyharnstoff.

17. Als Verbindung gemäss Anspruch 1 N-[4-(3'-Aethoxy-phenoxy)-phenyl]-N'-methyl-N'-methoxy-harnstoff.

18. Als Verbindung gemäss Anspruch 1 N-[4-(3'-Aethoxy-phenoxy)-phenyl]-N',N'-dimethylharnstoff.

19. Die Herstellung der Harnstoffe gemäss, Anspruch 1, dadurch gekennzeichnet, dass man ein Isocyanat der Formel II

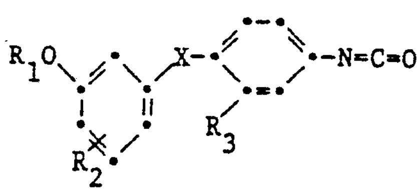

(II)

worin $R_1$, $R_2$, $R_3$ und X die unter Formel I im Anspruch 1 gegebene Bedeutung haben, in einem inerten organischen Lösungs- oder Verdünnungsmittels mit einem Amin der Formel III umsetzt,

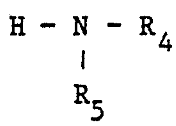

(III)

worin $R_4$ und $R_5$ die unter Formel I im Anspruch 1 gegebene Bedeutung haben.

20. Herbizides Mittel, dadurch gekennzeichnet, dass es neben inerten Zutaten als aktive Komponente mindestens einen Harnstoff der Formel I, Anspruch 1, enthält.

21. Die Verwendung der Harnstoffe gemäss, Anspruch 1, oder sie enthaltende Mittel zur Bekämpfung unerwünschten Pflanzenwachstums.

22. Die verwendung der Harnstoffe gemäss, Anspruch 1, oder sie enthaltende Mittel zur selektiven Bekämpfung von Unkräutern in Kulturen von Soja, Getreiden, Mais und Reis.

## Claims

1. A novel urea of the formula I

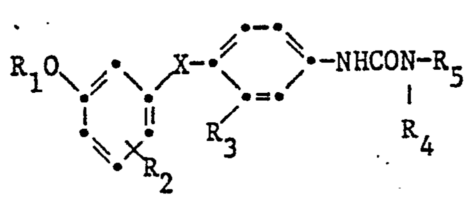

(I)

wherein

$R_1$ is a $C_1$-$C_6$-alkyl, $C_2$-$C_6$-alkenyl, $C_2$-$C_6$-alkynyl, aralkyl, phenyl, $C_1$-$C_6$-haloalkyl, $C_1$-$C_6$-cyanoalkyl, $C_2$-$C_6$-alkoxyalkyl, $C_2$-$C_6$-alkylthioalkyl, acyl, haloacyl or $C_1$-$C_4$-alkylsulfonyl group, or a group

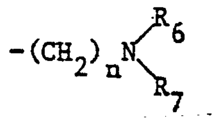 (I)

$R_2$ is hydrogen, halogen, $C_1$-$C_4$-alkyl or $C_1$-$C_4$-alkoxy,

$R_3$ is hydrogen, halogen, cyano or trifluoromethyl,

$R_4$ is hydrogen, $C_1$-$C_6$-alkyl, $C_2$-$C_6$-alkenyl, $C_2$-$C_6$-alkynyl or $C_1$-$C_6$-alkoxy,

$R_5$ is hydrogen, $C_1$-$C_6$-alkyl or $C_2$-$C_6$-alkenyl, or $R_5$ together with $R_4$ and the nitrogen atom to which they are bound form a 5-6-membered heterocycle,

$R_6$ and $R_7$ are each separately hydrogen, $C_1$-$C_6$-alkyl or $C_2$-$C_6$-alkenyl, or together with the nitrogen atom to which they are bound they also form a 5-6-membered heterocycle,

n is a number 1, 2 or 3, and

X is oxygen, sulfur or the sulfinyl or sulfonyl group.

2. A compound according to Claim 1, corresponding to formula I wherein X is oxygen, and $R_3$ is hydrogen, and $R_1$, $R_2$, $R_4$ and $R_5$ have the given meanings.

3. A compound according to Claim 1 of the formula Ia

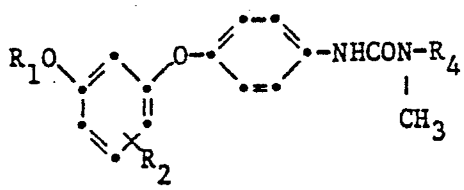 (Ia)

wherein

$R_1$ is a straight-chain or branched-chain $C_1$-$C_6$-alkyl group,

$R_2$ is hydrogen, halogen, methyl or methoxy, and

$R_4$ is methyl or methoxy.

4. A compound according to Claim 1 of the formula Ib

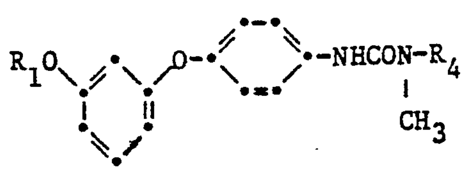 (Ib)

wherein

$R_1$ is a straight-chain or branched-chain $C_1$-$C_6$-alkyl group, and

$R_4$ is methyl or methoxy.

5. A compound according to Claim 1, corresponding to the formula I wherein X is sulfur, a sulfinyl or sulfonyl group, $R_2$ and $R_3$ are each hydrogen, and $R_1$, $R_4$ and $R_5$ have the given meanings.

6. As a compound according to Claim 1 : N-[4-(3′-methoxyphenoxy)-phenyl]-N′,N′-dimethylurea.

7. As a compound according to Claim 1 : N-[4-(3′-methoxyphenoxy)--phenyl]-N′-methyl-N′-methoxyurea.

8. As a compound according to Claim 1 : N-[3-chloro-4-(3′-methoxyphenoxy)-phenyl]-N′,N′-dimethylurea.

9. As a compound according to Claim 1 : N-[3-chloro-4-(3′-methoxyphenoxy)-phenyl]-N′-methyl-N′-methoxyurea.

10. As a compound according to Claim 1 : N-[4-(3′,5′-dimethoxyphenoxy)-phenyl]N′,N′-dimethylurea.

11. As a compound according to Claim 1 : N-[4-(3′,5′-dimethoxyphenoxy)-phenyl]-N′-methyl-N′-methoxyurea.

12. As a compound according to Claim 1 : N-[3-chloro-4-(4′-chloro-3′-methoxyphenoxy)-phenyl]-N′,N′-dimethylurea.

13. As a compound according to Claim 1 : N-[3-chloro-4-(4'-chloro-3'-methoxyphenoxy)-phenyl]-N'-methyl-N'-methoxyurea.

14. As a compound according to Claim 1 : N-[4-(3'-methoxy-6'-chlorophenoxy)-phenyl]-N',N'-dimethylurea.

15. As a compound according to Claim 1 : N-[4-(3'-propargyloxyphenoxy)-phenyl]-N',N'-dimethylurea.

16. As a compound according to Claim 1 : N-[4-(3'-propargyloxyphenoxy)-phenyl]-N'-methyl-N'-methoxyurea.

17. As a compound according to Claim 1 : N-[4-(3'-ethoxyphenoxy)-phenyl]-N'-methyl-N'-methoxyurea.

18. As a compound according to Claim 1 : N-[4-(3'-ethoxyphenoxy)-phenyl]-N',N'-dimethylurea.

19. The production of a urea according to Claim 1 whereby an isocyanate of the formula II

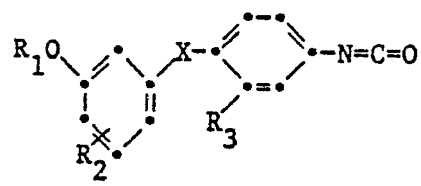

(II)

wherein $R_1$, $R_2$, $R_3$ and X have the meanings defined under the formula I in Claim 1, is reacted, in an inert organic solvent or diluent, with an amine of the formula III

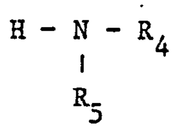

(III)

wherein $R_4$ and $R_5$ have the meanings defined under the formula I in Claim 1.

20. A herbicidal composition which contains as active ingredient at least one urea of the formula I, Claim 1, together with inert additives.

21. A method for combating undesirable plant growth which method comprises applying thereto or to the locus thereof a herbicidally effective amount of a urea according to Claim 1, or of a composition containing it.

22. A method for selectively combating weeds in cultivated crops of soya-bean, cereals, maize and rice, which method comprises applying thereto or to the locus thereof a herbicidally effective amount of a urea according to Claim 1, or of a composition containing it.

**Revendications**

1. Nouvelles urées de formule I

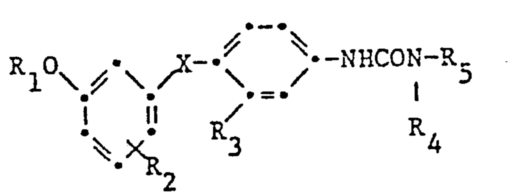

(I)

où

$R_1$ représente un radical alcoyle en $C_1$ à $C_6$, alcényle en $C_2$ à $C_6$, alcynyle en $C_2$ à $C_6$, aralcoyle, phényle, haloalcoyle en $C_1$ à $C_6$, cyanoalcoyle en $C_1$ à $C_6$, alcoxyalcoyle en $C_2$ à $C_6$, alcoylthioalcoyle en $C_2$ à $C_6$, acyle, haloacyle, un radical alcoyle en $C_1$ à $C_4$-sulfonyle ou un groupe

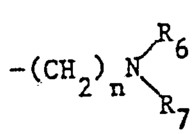

$R_2$ représente un hydrogène, un halogène, un alcoyle en $C_1$ à $C_4$ ou un alcoxy en $C_1$ à $C_4$,

$R_3$ représente un hydrogène, un halogène, un cyano ou un trifluorométhyle,

$R_4$ représente un hydrogène, un alcoyle en $C_1$ à $C_4$, un alcényle en $C_2$ à $C_6$, un alcynyle en $C_2$ à $C_6$ ou un alcoxy en $C_1$ à $C_6$,

$R_5$ représente un hydrogène, un alcoyle en $C_1$ à $C_6$, un alcényle en $C_2$ à $C_6$ ou encore avec $R_4$ et l'atome d'azote auquel ils sont liés un hétérocycle à 5 à 6 chaînons,

$R_6$ et $R_7$ représentent chacun isolément un hydrogène, un alcoyle en $C_1$ à $C_6$ ou un alcényle en $C_2$ à $C_6$ ou encore avec l'atome d'azote auquel ils sont liés un hétérocycle à 5 ou 6 chaînons,

n représente le nombre 1, 2 ou 3 et

X représente un oxygène, un soufre, le groupe sulfinyle ou le groupe sulfonyle.

2. Les composés selon la revendication 1 correspondant à la formule I, où X représente un oxygène et $R_3$ représente un hydrogène, tandis que $R_1$, $R_2$, $R_4$ et $R_5$ ont la signification donnée.

3. Les composés selon la revendication 1 de formule Ia

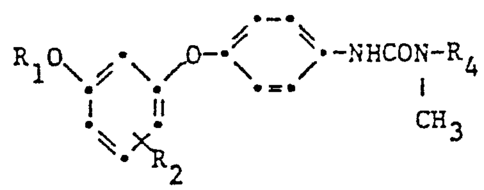

(Ia)

où

$R_1$ représente un radical alcoyle en $C_1$ à $C_6$ à chaîne droite ou ramifiée,

$R_2$ représente un hydrogène, un halogène, un méthyle ou un méthoxy, et

$R_4$ représente un méthyle ou un méthoxy.

4. Les composés selon la revendication 1 de formule Ib

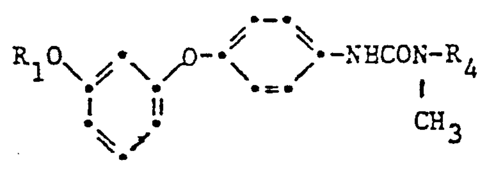

(Ib)

où

$R_1$ représente un radical alcoyle en $C_1$ à $C_6$ à chaîne droite ou ramifiée et

$R_4$ représente un méthyle ou un méthoxy.

5. Les composés selon la revendication 1 correspondant à la formule I, où X représente un soufre, un groupe sulfinyle ou sulfonyle, $R_2$ et $R_3$ représentent chacun un hydrogène, tandis que $R_1$, $R_4$ et $R_5$ ont la signification mentionnée.

6. Comme composé selon la revendication 1 la N-[4-(3'-méthoxy-phénoxy)-phényl]-N',N'-diméthylurée.

7. Comme composé selon la revendication 1 la N-[4-(3'-méthoxyphénoxy)-phényl]-N'-méthyl-N'-méthoxy-urée.

8. Comme composé selon la revendication 1 la N-[3-chloro-4-(3'-méthoxy-phénoxy)-phényl]-N',N'-diméthylurée.

9. Comme composé selon la revendication 1 la N-[3-chloro-4-(3'-méthoxyphénoxy)-phényl]-N'-méthyl-N'-méthoxy-urée.

10. Comme composé selon la revendication 1 la N-[4-(3',5'-diméthoxyphénoxy)-phényl]-N',N'-diméthylurée.

11. Comme composé selon la revendication 1 la N-[4-(3',5'-diméthoxyphénoxy)-phényl]-N'-méthyl-N'-méthoxy-urée.

12. Comme composé selon la revendication 1 la N-[3-chloro-4-(4'-chloro-3'-méthoxyphénoxy)-phényl]-N',N'-diméthylurée.

13. Comme composé selon la revendication 1 la N-[3-chloro-4-(4'-chloro-3'-méthoxyphénoxy)-phényl]-N'-méthyl-N'-méthoxy-urée.

14. Comme composé selon la revendication 1 la N-[4-(3'-méthoxy-6'-chlorophénoxy)-phényl]-N',N'-diméthylurée.

15. Comme composé selon la revendication 1 la N-[4-(3'-propargyloxyphénoxy)-phényl]-N',N'-diméthylurée.

16. Comme composé selon la revendication 1 la N-[4-(3'-propargyloxyphénoxy)-phényl]-N'-méthyl-N'-méthoxyurée.

17. Comme composé selon la revendication 1 la N-[4-(3'-éthoxy-phénoxy)-phényl]-N'-méthyl-N'-méthoxyurée.

18. Comme composé selon la revendication 1 la N-[4-(3'-éthoxy-phénoxy)-phényl]-N',N'-diméthylurée.

19. Préparation des urées selon la revendication 1, caractérisée en ce qu'on fait réagir un isocyanate de formule II

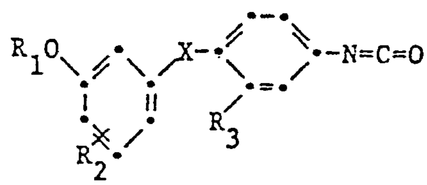

(II)

où $R_1$, $R_2$, $R_3$ et X ont la signification donnée dans la revendication 1, dans un solvant ou diluant organique, avec une amine de formule III

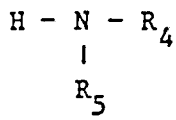

(III)

où $R_4$ et $R_5$ ont la signification donnée pour la formule I dans la revendication 1.

20. Agent herbicide caractérisé en ce qu'il contient outre des composants inertes comme composant actif au moins une urée de formule I de la revendication 1.

21. Application des urées selon la revendication 1 ou des agents qui les contiennent à la lutte contre la croissance végétale indésirable.

22. Application des urées selon la revendication 1 ou des agents qui les contiennent à la lutte sélective contre les mauvaises herbes dans les cultures de soja, de céréales, de maïs et de riz.